(19) Europäisches Patentamt
European Patent Office
Office européen des brevets

(11) Veröffentlichungsnummer: **0 003 958**
**B1**

(12) # EUROPÄISCHE PATENTSCHRIFT

(45) Veröffentlichungstag der Patentschrift: **17.02.82**

(51) Int. Cl.³: **G 01 N 33/48,** A 61 B 5/14

(21) Anmeldenummer: **79100090.4**

(22) Anmeldetag: **12.01.79**

(54) **Verfahren zur Analyse einer Messflüssigkeit in Abhängigkeit von einem Aufbereitungszustand einer in der Messflüssigkeit enthaltenen Probe.**

(30) Priorität: **14.03.78 CH 2735/78**

(43) Veröffentlichungstag der Anmeldung: **19.09.79 Patentblatt 79/19**

(45) Bekanntmachung des Hinweises auf die Patenterteilung: **17.02.82 Patentblatt 82/7**

(84) Benannte Vertragsstaaten: **BE CH DE FR GB IT NL SE**

(56) Entgegenhaltungen:
**DE-A-2 212 388**
**FR-A-2 186 780**
**FR-A-2 302 525**

(73) Patentinhaber: **CONTRAVES AG, Schaffhauserstrasse 580, CH-8052 Zürich (CH)**

(72) Erfinder: **Frey, Raymond, Dr., Schweigmatt 39, CH-8055 Zürich (CH)**

(74) Vertreter: **Köver, François, Dr. et al, Patentanwälte H.Mitscherlich K. Gunschmann, Dr. W. Körber, J.Schmidt-Evers Steinsdorfstrasse 10, D-8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 003 958 B1

# Verfahren zur Analyse einer Meßflüssigkeit in Abhängigkeit von einem Aufbereitungszustand einer in der Meßflüssigkeit enthaltenen Probe

Die Erfindung betrifft ein Verfahren zur Analyse einer Meßflüssigkeit in einem Analysensystem gemäß dem Oberbegriff des Anspruchs 1.

Die Probe ist beispielsweise — und wie in den Oberbegriffen der Ansprüche 2 und 3 präzisiert wird — eine Blutprobe. Bei der Untersuchung einer Blutprobe sind bekanntlich einer oder mehrere der folgenden Kennwerte zu messen: Konzentration der Erythrozyten, Konzentration der Leukozyten, Konzentration der Thrombozyten, Gewichtskonzentration an Hämoglobin, mittleres Volumen der Erythrozyten, Volumenanteil der Erythrozyten in der Blutprobe (Hämatokritwert), mittleres Gewicht des Hämoglobins in einem Erythrozyten, und eventuell noch andere Kennwerte. Die Probe kann beispielsweise auch aus anderen biologischen Stoffen wie Urin, Galle, Lymphe, Plasma bestehen, in denen verschiedene Komponenten wie Farbstoffe, Zucker, Eiweiß usw. als Kennwerte zu bestimmten sind, Es kann sich auch um Bakterienkolonien, Abwasserproben, Staubproben handeln, in denen die Konzentration von Stoffen und/oder die Verteilung von Teilchen zu messen sind. Schließlich kann die Probe eine Leseprobe oder eine Standardprobe zu Eichzwecken sein.

Zur Durchführung solcher Analysen werden solche Proben zumeist einer Trägerflüssigkeit — z. B. Wasser oder isotonische Plasmaersatzlösung — in vorbestimmter Verdünnung beigefügt, wobei nach Bedarf auch eine chemische oder biologische Behandlung — z. B. Oxydation, Reduktion, Hämolyse usw. — oder noch eine Markierung — z. B. Fluoreszenz oder Radioaktivität — mittels Zusatz einer Markierungssubstanz vorgenommen wird. Derartige Behandlungen der Probe vor oder nach deren Verdünnung in der Trägerflüssigkeit sowie das Erreichen des gewünschten Lösungs- oder Suspensionseffektes beim gewünschten Verdünnungsgrad werden unter dem Begriff »Aufbereitung der Probe« zusammengefaßt, und der Zustand, in welchem die Probe in der Trägerflüssigkeit schließlich enthalten ist, wird als »Aufbereitungszustand« bezeichnet.

Analysensysteme der eingangs genannten Art sind beispielsweise aus den DE-C-1 673 146, DE-C-1 798 431 und DE-C-2 324 057 bekannt. In derartigen Analysensystemen werden mehrere Kennwerte mittels mehrerer Analysiervorrichtungen bestimmt, jede Analysiervorrichtung (bestehend u. a. aus Sensor und Auswertevorrichtung) ist aber auf einen vorbestimmten Aufbereitungszustand abgestimmt, wobei nicht von Belang ist, ob diese Aufbereitung von Hand oder (z. B. in DE-C-1 798 431) vom Gerät selbst vorgenommen wird. Für eine automatische Feststellung, daß zwecks Eichung dieser Geräte reine Trägerflüssigkeit analysiert wird, ist nichts vorgesehen. Auch ist es damit nicht möglich, dieselbe Analysiervorrichtung wahlweise zur Zählung von Erythrozyten, von Leukozyten oder von Thrombozyten zu verwenden und damit Kosten zu sparen: die Analysiervorrichtungen — in Kombination mit den daran angeschlossenen Rechenvorrichtungen — liefern falsche Werte der Kennwerte, wenn andere als die vorgesehenen Aufbereitungszustände verwendet werden.

Eine Analyse der reinen Trägerflüssigkeit ist beispielsweise in der DE-B-2 058 081 vorgeschlagen worden: in einer zusätzlichen und dafür vorgesehenen Analysiervorrichtung wird reine Trägerflüssigkeit laufend gemessen, um einen in Errechnung eines Kennwertes verwendeten Koeffizienten den Veränderungen der Eigenschaften der reinen Trägerflüssigkeit anzupassen. Nichtsdestoweniger werden auch in diesem Analysensystem falsche Werte geliefert, wenn andere als der einzige vorgesehene Aufbereitungszustand verwendet werden.

Verfahren zur Feststellung von falschen Meßwerten sind beispielsweise in der DE-C-2 116 595 und DE-C-2 120 697 vorgeschlagen worden: hier handelt es sich jedoch nur um die Feststellung des fehlerhaften Funktionierens einer Analysiervorrichtung; es besteht kein Zusammenhang mit dem Aufbereitungszustand der Probe. Übrigens ist das Vergleichen von Meßwerten mit Schwellenwerten in Analysensystemen an sich bekannt; außer zur Feststellung von Fehlfunktionen wird es auch zum Klassieren von Meßwerten zur Erstellung eines Histogrammes verwendet, wie beispielsweise aus der DE-A-2 418 559 hervorgeht.

Eine selbstanpassende Bestimmung eines Kennwertes aufgrund des Aufbereitungszustandes der Probe wurde beispielsweise in der DE-A-2 166 597 vorgeschlagen. In diesem Analysensystem werden die zur Deutung der Analysenergebnisse erforderlichen Koeffizienten selbsttätig bereitgestellt Zu diesem Zweck werden Flüssigkeitsbehälter verwendet, die mit besonderen Codierungsmitteln versehen sind. Je einem bestimmten Aufbereitungszustand entspricht je ein Codezeichen derart, daß das Einführen des Flüssigkeitsbehälters in das Analysengerät das System auf eine dem Codezeichen entsprechende Errechnung des Kennwertes einstellt. Allerdings ist das Analysensystem selbstanpassend in bezug auf das Codezeichen, das vom Flüssigkeitsbehälter getragen wird, und nicht in bezug auf die Flüssigkeit, die im Behälter enthalten ist. Das Bedienungspersonal muß also darauf achten, entsprechend dem Aufbereitungszuständen jeweils den richtigen Flüssigkeitsbehälter zu verwenden; das Problem des selbstanpassenden Analysensystems wurde also in der zitierten Druckschrift erkannt, die vorgeschlagene Lösung kann aber nicht als selbstanpassend bezeichnet werden.

Die Selbstanpassung des Analysensystems braucht, wie beispielsweise in der DE-A-2 529 902 vorgeschlagen wurde, sich nicht nur auf die Wahl der geeigneten Koeffizienten zu beschränken, vielmehr kann sie sich auf die Wahl des gesamten geeigneten Meß- und Rechenprogramms beziehen. Auch hier wird aber die Selbstanpassung durch mechanischoptische Mittel gesteuert, welche den die Proben enthaltenden Flüssigkeitsbehältern durch das Bedienungspersonal von Hand zugeordnet werden, so daß auch diese Lösung die bereits erwähnten Nachteile aufweist und nicht als selbstanpassend bezeichnet werden kann.

Der Erfindung liegt die Aufgabe zugrunde, in einem Analysensystem der eingangs erwähnten Art ein Verfahren zu schaffen, um die Bestimmung eines Kennwertes selbsttätig, d. h. unmittelbar und insbesondere ohne Dazutun einer Bedienungsperson, an den Aufbereitungszustand der Probe anzupassen. Der Lösung dieser Aufgabe liegt folgende Erkenntnis zugrunde: im Ablauf der Analyse ist vorerst festzustellen, in welchem Aufbereitungszustand die Probe vorliegt; erst dann ist es möglich, den oder die Kennwerte zu errechnen; der Aufbereitungszustand ist anhand eines oder mehrerer Meßwerte feststellbar, wobei es nicht erforderlich ist, daß es dieselben Meßwerte sind, die zur Errechnung des oder der Kennwerte herangezogen wurden.

Beispielsweise ist die Thrombozytenkonzentration in Blut zu bestimmen, wobei die Aufbereitung der Blutprobe im wesentlichen entweder darin besteht, das Blut in isotonischer Plasmaersatzlösung im Verhältnis 1 : 80 000 zu verdünnen, oder darin besteht, das Blut zu zentrifugieren und das im Überstand erhaltene Plasma in isotonischer Plasmaersatzlösung im Verhältnis 1 : 2200 zu verdünnen. Zur Bestimmung der Thrombozytenkonzentration wird die Anzahl Thrombozyten in beispielsweise 200 µl probenhaltiger Meßflüssigkeit gemessen, und der erhaltene Meßwert wird — je nach der verwendeten Aufbereitung — mit 400 oder mit 11 multipliziert, um den gewünschten Kennwert in einer der Fachwelt üblichen Einheit (Anzahl Teilchen pro µl) auszudrücken. Nun ist ja bekannt, daß im menschlichen Blut normalerweise 150 000 bis 350 000 Thrombozyten pro µl enthalten sind: je nach der verwendeten Aufbereitung wird der Meßwert normalerweise in der Größenordnung von 600 oder von 20 000 liegen. Ein Schwellenwert kann also bei 4000 gesetzt werden; durch Vergleich mit diesem Schwellenwert lassen sich Meßwerte von beispielsweise 1000 bzw. 10 000 ohne weiteres einem der beiden Aufbereitungszustände zuordnen, obschon solche Meßwerte durchaus pathologischen Thrombozytenkonzentrationen von 400 000 bzw. 110 000 $\mu l^{-1}$ entsprechen — die alternative Zuordnung ergäbe die nicht wahrscheinlichen Thrombozytenkonzentrationen von 11 000 bzw. 4 000 000 $\mu l^{-1}$. Zweifelsfälle ergeben sich aber mit Meßwerten von beispielsweise 5000: die entsprechend resultierende Thrombozytenkonzentration beträgt im einen der Aufbereitungszustände 2 000 000 $\mu l^{-1}$, im anderen Aufbereitungszustand 55 000 $\mu l^{-1}$, und diese beiden Thrombozytenkonzentrationen sind zwar stark pathologisch, jedoch beide noch möglich, wenn auch wenig wahrscheinlich. Hier erlaubt der Vergleich zwischen dem Meßwert und dem Schwellenwert nicht, zwischen den beiden möglichen Aufbereitungszuständen der Probe zu unterscheiden: es muß ein weiteres Unterscheidungsmerkmal herangezogen weden, das beispielsweise die Erythrozytenkonzentration sein kann. Eine sehr niedrige Erythrozytenkonzentration läßt darauf schließen, daß die Probe Plasma ist und demzufolge im Verdünnungsverhältnis 1 : 2200 vorliegt, während ein Meßwert der Erythrozytenzählung, der einer physiologisch erreichbaren Erythrozytenkonzentration entspricht, darauf deutet, daß die Probe Blut ist und folglich im Verdünnungsverhältnis 1 : 80 000 vorliegt.

Entsprechend diesem erkannten Lösungsweg ist das erfindungsgemäße Verfahren gekennzeichnet durch die im Anspruch 1 aufgeführten Verfahrensschritte. Vorteilhafte Ausführungsweisen ergeben sich aus den Ansprüchen 2 und 3. Ein Zusatzverfahren zur Auslösung einer Eichung wird im Anspruch 4, eine weitere vorteilhafte Ausführungsweise im Anspruch 5 vorgeschlagen.

Im folgenden wird die Erfindung unter Bezugnahme auf die Zeichnung näher beschrieben. Es zeigt

Fig. 1 eine schematische Darstellung eines hämatologischen Analysensystems zur Durchführung eines ersten Beispiels des Verfahrens,

Fig. 2 eine schematische Darstellung eines anderen hämatologischen Analysensystems zur Durchführung eines zweiten Beispiels des Verfahrens und

Fig. 3 eine schematische Darstellung einer verallgemeinerten Form des Verfahrensablaufes.

Zur Erläuterung eines ersten Beispiels des Verfahrens ist in Fig. 1 ein hämatologisches Analysensystem schematisch dargestellt. Die von diesem System zu analysierende Meßflüssigkeit besteht, in einem ersten Aufbereitungszustand, aus einer Blutprobe, die in isotonischer Plasmaersatzlösung — beispielsweise in einer Lösung von 0,9% Natriumchlorid in Wasser — im Verhältnis 1 : 80 000 verdünnt wird. In einem zweiten Aufbereitungszustand besteht die Meßflüssigkeit aus einer Blutprobe, die in isotonischer Plasmaersatzlösung im Verhältnis 1 : 400 verdünnt wird, wobei ein Hämolysiermittel — beispielsweise Trimethyl-hexadecylammoniumchlorid — beigegeben wird. In einem Eichzustand besteht die Meßflüssigkeit aus reiner isotonischer Plasmaersatzlösung. Welcher Aufbereitungszustand vorliegt bzw. ob der Eichzustand vorliegt, braucht zu Beginn der Messung dem Analysensystem nicht eingegeben zu werden: das Analysensystem bestimmt dies selbsttätig, wie im folgenden noch gezeigt wird.

Im Analysensystem ist mit 1 eine Meßvorrichtung zur Zählung der in der Meßflüssigkeit

suspendierten Teilchen bezeichnet; es handelt sich beispielsweise um einen Teilchenzähler der in der DE-C-2 121 201 beschriebenen Art, welcher zur Zählung sowohl von Erythrozyten als auch von Leukozyten ausgebildet ist. An einem Ausgang 11 des Teilchenzählers 1 wird ein Meßwert geliefert, der aus einem digitalen Zählergebnis besteht.

Mit 2 ist eine Meßvorrichtung zur fotometrischen Messung eines Hämoglobingehaltes in der Meßflüssigkeit bezeichnet; es handelt sich beispielsweise um ein Fotometer der in der DE-C-2 535 128 beschriebenen Art, welches zur Bestimmung des gesamten Gehaltes der Meßflüssigkeit an verschiedenen Hämoglobinderivaten (beispielsweise Hb, $HbO_2$, HbCO und Hi) ausgebildet ist. An einem Ausgang 21 des Fotometers 2 wird ein Meßwert geliefert, welcher der Konzentration des vorhandenen Hämoglobins entspricht und entweder in digitaler Form oder in analoger Form vorliegen kann.

Mit 3 ist eine Meßvorrichtung zur Messung der Leitfähigkeit der Meßflüssigkeit bezeichnet; es handelt sich beispielsweise um ein Gerät der in der US-A-2 871 445 beschriebenen Art. An einem Ausgang 31 des Leitfähigkeits-Meßgerätes 3 wird ein Meßwert geliefert, welcher der Leitfähigkeit der Meßflüssigkeit entspricht und entweder in digitaler Form oder in analoger Form vorliegen kann. Von den Ausgängen 11, 21, 31 werden die Meßwerte aus den entsprechenden Meßvorrichtungen 1, 2, 3 über entsprechende Leitungen je einem Vergleicher 12, 22, 32 zugeführt. In jedem dieser Vergleicher wird der ihm zugeführte Meßwert mit einem zugeordneten und vorbestimmten Schwellenwert verglichen, der gleich 10% der unteren Grenze des beim menschlichen Blut zu erwartenden Meßwertes gesetzt wird. Bekanntlich liegt beim menschlichen Blut die Erythrozytenkonzentration etwa zwischen 4 und 6 Millionen Teilchen pro µl, die Leukozytenkonzentration etwa zwischen 5000 und 11 000 Teilchen pro µl, die Hämoglobinkonzentration etwa zwischen 120 und 180 g/l, während die spezifische Leitfähigkeit der als Trägerflüssigkeit in den beiden Aufbereitungszuständen sowie im Eichzustand verwendeten gebräuchlichen isotonischen Plasmaersatzlösungen etwa zwischen 1,5 und 2 $Ohm^{-1} \cdot m^{-1}$ liegt. Dementsprechend wird der Schwellenwert des Vergleichers 12 gleich dem Meßwert gesetzt, der von der Meßvorrichtung 1 geliefert würde, wenn diese eine 5 Teilchen pro µl enthaltende Meßflüssigkeit messen würde (erster Aufbereitungszustand); der Schwellenwert des Vergleichers 22 wird gleich dem Meßwert gesetzt, der von der Meßvorrichtung 2 geliefert würde, wenn diese eine 30 mg/l Hämoglobin enthaltende Meßflüssigkeit messen würde (zweiter Aufbereitungszustand); und der Schwellenwert des Vergleichers 32 wird gleich dem Meßwert gesetzt, der von der Meßvorrichtung 3 geliefert würde, wenn diese eine Meßflüssigkeit mit einer spezifischen Leitfähigkeit von 0,15 $Ohm^{-1} \cdot m^{-1}$ messen würde (dies schließt u. a. Reinigungsflüssigkeit aus). Derartige Vergleicher zum Vergleichen eines zugeführten variablen Meßwertes mit einem vorbestimmten Schwellenwert sind bekannt, und zwar für digitale wie auch für analoge Werte. An je einem Ausgang 13, 23, 33 der Vergleicher 12, 22, 32 erscheint je ein logisches Signal, das 1 ist, wenn der Meßwert größer ist als der Schwellenwert, und das im gegenteiligen Fall 0 ist.

Einer UND-Torschaltung 101 werden eingangsseitig die Signale aus den Ausgängen 13, 23, 33 über entsprechende Leitungen zugeführt, wobei zur logischen Umkehr des Signals aus dem Ausgang 23 dieses Signal über einen Invertierer 24 geführt wird. Einer UND-Torschaltung 102 werden eingangsseitig die Signale aus den Ausgängen 23 und 33 über entsprechende Leitungen zugeführt. Einer UND-Torschaltung 103 werden eingangsseitig die Signale aus den Ausgängen 13, 23, 33 über entsprechende Leitungen zugeführt, wobei zur logischen Umkehr der Signale aus den Ausgängen 13 und 23 das erstgenannte Signal über einen Invertierer 14 und das zweitgenannte Signal über den Invertierer 24 geführt wird.

Der Meßwert aus dem Ausgang 11 der Meßvorrichtung 1 wird einem Dateneingang 113 eines digitalen Rechners 110 über eine entsprechende Leitung zugeführt. Die Ausgangssignale der UND-Torschaltungen 101 und 102 werden je einem Steuereingang 111 und 112 des Rechners 110 über entsprechende Leitungen zugeführt. Der Meßwert aus dem Ausgang 21 der Meßvorrichtung 2 wird einem Dateneingang 123 eines Rechners 120 über eine entsprechende Leitung zugeführt; entsprechend der Art des Meßwertes am Ausgang 21 ist der Rechner 120 ein digitaler oder analoger Rechner, für einen analogen Meßwert kann es auch ein digitaler Rechner sein, der mit einem Analog-Digital-Wandler zum Umsetzen des Meßwertes in digitale Form versehen ist. Das Ausgangssignal der UND-Torschaltung 102 wird einem Steuereingang122 des Rechners 120 über eine entsprechende Leitung zugeführt. Schließlich wird das Ausgangssignal der UND-Torschaltung 103 je einem Steuereingang 114 und 124 der Rechner 110 und 120 über entsprechende Leitungen zugeführt.

Das im Zusammenhang mit Fig. 1 beschriebene Analysensystem ermöglicht den folgenden Verfahrensablauf: Zunächst wird die zu analysierende Meßflüssigkeit gleichzeitig den drei Meßvorrichtungen 1, 2 und 3 zugeführt, worauf an den Ausgängen 11, 21 und 31 dieser Meßvorrichtungen Meßwerte erscheinen. Falls die Meßflüssigkeit den drei Meßvorrichtungen nacheinander zugeführt wird, sind beispielsweise an den Ausgängen 11, 21, 31 Zwischenspeicher vorzusehen, die auf bekannte Weise vor Anfang der Analyse rückgestellt werden und während der Analyse die Meßwerte speichern und halten. Der Meßwert am Ausgang 11 dient als erster Hilfswert, der Meßwert am Ausgang 21 dient als zweiter Hilfswert, der Meßwert am Ausgang 31 dient als Eichungs-Hilfswert. In den

Vergleichern 12, 22, 32 werden diese Hilfswerte mit den zugeordneten Schwellenwerten verglichen; das logische Signal, das als Ergebnis des Vergleichers am Ausgang 13 des Vergleichers 12 ansteht, dient als entsprechendes erstes Erkennungssignal; das logische Signal, das als Ergebnis des Vergleichers am Ausgang 23 des Vergleichers 22 ansteht, dient als entsprechendes zweites Erkennungssignal; und das logische Signal, das als Ergebnis des Vergleichers am Ausgang 33 des Vergleichers 32 ansteht, dient als entsprechendes Eichungssignal.

Die UND-Torschaltung 101, 102, 103 liefern bekanntlich ein logisches Ausgangssignal 1, wenn auf allen ihren jeweiligen Eingängen je ein logisches Signal 1 ansteht: diese UND-Torschaltungen dienen also als Vergleicher zwischen einem Sollzustand 1,1,1 und der Kombination der jeweiligen, ihren Eingängen zugeführten Istzuständen.

Zufolge der Wirkung des Invertierers 24 liefert die UND-Torschaltung 101 ein Ausgangssignal 1, wenn gleichzeitig am Ausgang 13 ein erstes Erkennungssignal mit einem Istzustand 1, am Ausgang 23 ein zweites Erkennungssignal mit einem Istzustand 0 und am Ausgang 33 ein Eichungssignal mit einem Istzustand 1 anstehen. Folglich ist die Wirkung dieser Kombination des Invertierers 24, der UND-Torschaltung 101 und der entsprechenden Verbindungen, das erste Erkennungssignal, das zweite Erkennungssignal und das Eichungssignal in dieser Reihenfolge zu einem Erkennungs-Datenwort zusammenzufassen, dieses mit einem ersten Aufbereitungs-Datenwort 1,0,1 (entsprechend derselben Reihenfolge) zu vergleichen, und bei Übereinstimmung ein erstes logisches Steuersignal 1 dem Eingang 11 des Rechners 110 zuzuführen. Auf ähnliche Weise ist die Wirkung der UND-Torschaltung 102 und der entsprechenden Verbindungen, dasselbe Erkennungs-Datenwort zu bilden, es mit je einem der zweiten Aufbereitungs-Datenwörter 1,1,1 oder 0,1,1 zu vergleichen, und bei Übereinstimmung mit einem dieser Aufbereitungs-Datenwörter ein zweites logisches Steuersignal 1 den Eingängen 112 und 122 der Rechner 110 und 120 zuzuführen. Auf ähnliche Weise ist die Wirkung der UND-Torschaltung 103 und der entsprechenden Verbindungen, dasselbe Erkennungs-Datenwort mit einem Eichungs-Datenwort 0,0,1 zu vergleichen, und bei Übereinstimmung ein den Eichungsvorgang bezeichnendes logisches Steuersignal 1 den Eingängen 114 und 124 der Rechner 110 und 120 zuzuführen.

Wie leicht ersichtlich ist, bedeutet das Erscheinen des ersten Steuersignals, daß in der Meßflüssigkeit viele Teilchen und hohe Leitfähigkeit, jedoch wenig Hämoglobin vorhanden sind: dies entspricht also dem ersten Aufbereitungszustand (Blutverdünnung 1 : 80 000). Das Erscheinen des zweiten Steuersignals bedeutet, daß viel Hämoglobin und hohe Leitfähigkeit vorhanden sind (die Anzahl Teilchen ist beliebig): dies entspricht also dem zweiten Aufbereitungszustand (Blutverdünnung 1 : 400 mit Hämolyse). Das Erscheinen des den Eichungsvorgang bezeichnenden Steuersignals bedeutet, daß wenig Teilchen und wenig Hämoglobin, jedoch hohe Leitfähigkeit vorhanden sind: dies entspricht also dem Eichzustand, in welchem die Meßflüssigkeit aus reiner isotonischer Plasmaersatzlösung besteht.

Der Rechner 110 ist auf dem Fachmann naheliegende Weise zur Ausübung der folgenden Funktionen ausgebildet oder programmiert. Zunächst wird geeicht. Bei Erhalt des den Eichungsvorgang bezeichnenden Steuersignals auf dem Steuereingang 114 wird der auf dem Dateneingang 113 erhaltene Meßwert (der in der Regel von Verunreinigungen herrührt) gespeichert, um als Basiswert von später erhaltenen, Blutproben entsprechenden, Meßwerten abgezogen zu werden. Bei Erhalt des ersten Steuersignals auf dem Steuereingang 111 wird von dem auf dem Dateneingang 113 erhaltenen Meßwert der gespeicherte Basiswert abgezogen, worauf die erhaltene Differenz mit einem Koeffizienten multipliziert wird, der dem Betrieb der Meßvorrichtung 1 und dem Verdünnungsverhältnis 1 : 80 000 entspricht und das Resultat der Teilchenzählung in Anzahl Teilchen pro µl Blut umsetzt; das in dieser Einheit ausgedrückte Resultat wird angezeigt oder gedruckt. Bei Erhalt des zweiten Steuersignals auf dem Steuereingang 112 erfolgt derselbe Vorgang, mit dem Unterschied, daß der Koeffizient nun dem Verdünnungsverhältnis 1 : 400 entspricht.

Der Rechner 120 ist ebenfalls auf dem Fachmann naheliegende Weise zur Ausübung der folgenden Funktionen ausgebildet oder programmiert. Bei Erhalt des den Eichungsvorgang bezeichnenden Steuersignals auf dem Steuereingang 124 wird der auf dem Dateneingang 123 erhaltene Meßwert gespeichert, um als Basiswert von später erhaltenen, Blutproben entsprechenden Meßwerten abgezogen zu werden. Bei Erhalt des zweiten Steuersignals auf dem Steuereingang 122 wird von dem auf dem Dateneingang 123 erhaltenen Meßwert der gespeicherte Basiswert abgezogen, worauf die erhaltene Differenz mit einem Koeffizienten multipliziert wird, der dem Betrieb der Meßvorrichtung 2 und dem Verdünnungsverhältnis 1 : 400 entspricht und das Resultat der Hämoglobinmessung in g/l Blut umsetzt. Das in dieser Einheit ausgedrückte Resultat wird angezeigt oder gedruckt.

Beim ersten Aufbereitungszustand werden alle Teilchen gezählt, so daß als Resultat die Summe der Konzentrationen an Erythrozyten und Leukozyten im Blut angegeben wird; da aber die Anzahl Leukozyten etwa im Verhältnis 1 : 1000 zur Anzahl Erythrozyten steht, ist der dadurch entstehende systematische Meßfehler vernachlässigbar. Beim zweiten Aufbereitungszustand sind die Erythrozyten hämolysiert, so daß dieser systematische Meßfehler nicht auftritt; es werden nur die Leukozyten gezählt.

Das beschriebene Verfahren kann auch mit anderen Vorrichtungen durchgeführt werden.

Unter anderen Möglichkeiten können die beiden Rechner 110 und 120 zu einer einzigen Recheneinheit zusammengefaßt werden, welche auch die UND-Torschaltungen 101, 102, 103 und die Invertierer 14, 24 umfaßt oder deren Funktionen ausübt. Wenn die Meßwerte an den Ausgängen 11, 21, 31, alle in digitaler Form vorliegen, ist es auch möglich, die Funktionen der Vergleicher 12, 22, 32 in die Recheneinheit aufzunehmen, so daß die drei Meßwerte direkt einer entsprechend programmierten Recheneinheit zugeführt werden; das Verfahren wird dann in dieser Recheneinheit durchgeführt.

Zur Erläuterung eines zweiten Beispiels des Verfahrens ist in Fig. 2 ein hämatologisches Analysensystem zur Thrombozytenzählung schematisch dargestellt. Die von diesem System zu analysierende Meßflüssigkeit besteht, in einem ersten Aufbereitungszustand, aus einer Blutprobe, die in isotonischer Plasmaersatzlösung im Verhältnis 1 : 80 000 verdünnt wird. In einem zweiten Aufbereitungszustand besteht die Meßflüssigkeit aus einer Blutprobe, aus der vorerst durch Zentrifugation ein erythrozytenarmes Plasma gewonnen wird, das anschließend in isotonischer Plasmaersatzlösung im Verhältnis 1 : 2200 verdünnt wird. In einem Eichzustand besteht die Meßflüssigkeit aus reiner isotonischer Plasmaersatzlösung. Das dabei zu lösende Problem der Bestimmung des vorliegenden Aufbereitungszustandes wurde im vorangehenden bereits erörtert.

In diesem Analysensystem ist mit 4 eine Meßvorrichtung zum Analysieren der in der Meßflüssigkeit suspendierten Teilchen auf deren Größenklasse und Anzahl pro Größenklasse bezeichnet; es handelt sich beispielsweise um einen Teilchenanalysator mit einem Meßkopf der in der DE-C-2 121 201 beschriebenen Art und einem bekannten Vielkanal-Impulshöhenanalysator, der hier als Zweikanal-Analysator betrieben wird. An einem Ausgang 41 wird ein Meßwert geliefert, der der Anzahl Thrombozyten in der Meßflüssigkeit entspricht: an einem Ausgang 51 wird ein Meßwert geliefert, der der Anzahl Erythrozyten in der Meßflüssigkeit entspricht; beide Meßwerte liegen als digitales Zählergebnis vor.

Mit 7 ist eine Meßvorrichtung zur Messung der Leitfähigkeit der Meßflüssigkeit bezeichnet: es handelt sich beispielsweise um ein Gerät der in der US-A-2 871 445 beschriebenen Art. An einem Ausgang 71 dieses Leitfähigkeits-Meßgerätes 7 wird ein Meßwert geliefert, welcher der Leitfähigkeit in der Meßflüssigkeit entspricht und entweder in digitaler Form oder in analoger Form vorliegen kann.

Von den Ausgängen 41 und 51 werden die Meßwerte aus der Meßvorrichtung 4 einerseits zu einem Quotientenbildner 410 und andererseits zu einem Addierwerk 610 über entsprechende Leitungen zugeführt. An einem Ausgang 41 des Quotientenbildners 410 wird ein digitaler Quotient geliefert, der gleich dem Verhältnis des Meßwertes für Erythrozyten zum Meßwert für Thrombozyten ist. An einem Ausgang 611 des Addierwerkes 610 wird ein digitaler Summenwert geliefert, der gleich der Summe des Meßwertes für Erythrozyten und des Meßwertes für Thrombozyten ist.

Von den Ausgängen 411, 51, 611 werden der Quotient, der Meßwert für Erythrozyten und der Summenwert über entsprechende Leitungen je einem Vergleicher 42, 52, 62 zugeführt. In jedem dieser Vergleicher wird der ihm zugeführte Wert mit einem zugeordneten und vorbestimmten Schwellenwert verglichen. Der Schwellenwert des Vergleichers 42 wird gleich 1 (=10% der unteren Grenze des beim menschlichen Blut zu erwartenden Wertes) gesetzt. Für den Schwellenwert des Vergleichers 52 ist zu berücksichtigen, daß auch im erythrozytenarmen Plasma etwa 10 000 bis 30 000 restliche Erythrozyten pro µl enthalten sind: beim ersten Aufbereitungszustand liegt demnach die untere Grenze der Erythrozytenkonzentration in der Meßflüssigkeit bei etwa 50 Teilchen pro µl, beim zweiten Aufbereitungszustand liegt die obere Grenze der Erythrozytenkonzentration bei etwa 15 Teilchen pro µl, so daß der Schwellenwert des Vergleichers 52 gleich dem Meßwert für die Anzahl Erythrozyten gesetzt wird, der von dem entsprechenden Ausgang 51 der Meßvorrichtung 4 geliefert würde, wenn diese eine 30 Teilchen pro µl enthaltende Meßflüssigkeit messen würde. Der Schwellenwert des Vergleichers 62 wird so tief wie möglich gesetzt, wobei die untere Grenze durch die Konzentration der in der reinen Trägerflüssigkeit schwebenden, störenden Teilchen gegeben wird: ein angemessener Schwellenwert liegt bei 5 Teilchen pro µl Meßflüssigkeit. Vom Ausgang 71 wird der Meßwert für die Leitfähigkeit über eine entsprechende Leitung einem Vergleicher 72 zugeführt, in welchem dieser Meßwert mit einem zugeordneten und vorbestimmten Schwellenwert verglichen wird, der gleich dem Meßwert für eine Meßflüssigkeit mit einer spezifischen Leitfähigkeit von 0,15 $Ohm^{-1} \cdot m^{-1}$ gesetzt wird, wie bereits im Zusammenhang mit Fig. 1 erläutert wurde.

An je einem Ausgang 43, 53, 63, 73 der Vergleicher 42, 52, 62, 72 erscheint je eine logisches Signal, das 1 ist, wenn der dem Vergleicher aus den Ausgängen 411, 51, 611, 72 zugeführte Wert größer ist als der Schwellenwert, und das im gegenteiligen Fall 0 ist.

Einer UND-Torschaltung 104 werden eingangsseitig die Signale aus den Ausgängen 45, 63, 73 über entsprechende Leitungen zugeführt. Einer UND-Torschaltung 106 werden eingangsseitig die Signale aus den Ausgängen 43, 53, 63, 73 über entsprechende Leitungen zugeführt, wobei zur logischen Umkehr der Signale aus den Ausgängen 43 und 53 diese Signale über je einen Invertierer 44 und 54 geführt werden. Einer UND-Torschaltung 107 werden eingangsseitig die Signale aus den Ausgängen 63, 73 über entsprechende Leitungen zugeführt, wobei zur logischen Umkehr des Signals aus dem Ausgang 63 dieses Signal über einen Invertierer 64 geführt

wird.

Der Meßwert aus dem Ausgang 41 der Meßvorrichtung 4 wird einem Dateneingang 143 eines digitalen Rechners 140 über eine entsprechende Leitung zugeführt. Die Ausgangssignale der UND-Torschaltungen 104, 106, 107 werden je einem Steuereingang 144, 146, 147 des Rechners 140 über entsprechende Leitungen zugeführt.

Das im Zusammenhang mit Fig. 2 beschriebene Analysensystem ermöglicht den folgenden Verfahrensablauf: Zunächst wird die zu analysierende Meßflüssigkeit gleichzeitig den zwei Meßvorrichtungen 4 und 7 zugeführt, worauf an den Ausgängen 41, 51 und 71 dieser Meßvorrichtungen Meßwerte erscheinen. Falls die Meßflüssigkeit den beiden Meßvorrichtungen nacheinander zugeführt wird, sind beispielsweise an den Ausgängen 41, 51, 71 Zwischenspeicher vorzusehen, die auf bekannte Weise vor Anfang der Analyse rückgestellt werden und während der Analyse die Meßwerte speichern und halten. Der im Quotientenbildner 410 gebildete Quotient des Meßwertes am Ausgang 51 zum Meßwert am Ausgang 41 dient als erster Hilfswert, der Meßwert am Ausgang 51 dient als zweiter Hilfswert, der im Addierer 610 gebildete Summenwert der Meßwerte an den Ausgängen 41 und 51 dient als dritter Hilfswert, der Meßwert am Ausgang 71 dient als Eichungs-Hilfswert. In den Vergleichern 42, 52, 62, 72 werden diese Hilfswerte mit den zugeordneten Schwellenwerten verglichen; das logische Signal, das als Ergebnis des Vergleiches am Ausgang 43 des Vergleichers 42 ansteht, dient als entsprechendes erstes Erkennungssignal; das logische Signal, das als Ergebnis des Vergleichers am Ausgang 53 des Vergleichers 52 ansteht, dient als entsprechendes zweites Erkennungssignal; das logische Signal, das als Ergebnis des Ausgangs 63 des Vergleichers 62 ansteht, dient als entsprechendes drittes Erkennungssignal: und das logische Signal, das als Ergebnis des Vergleichers am Ausgang 73 des Vergleichers 72 ansteht, dient als entsprechendes Eichungssignal.

Wie im Zusammenhang mit Fig. 1 beschrieben wurde, ist die Wirkung der UND-Torschaltung 104 und der entsprechenden Verbindungen, das erste Erkennungssignal, das zweite Erkennungssignal, das dritte Erkennungssignal und das Eichungssignal in dieser Reihenfolge zu einem Erkennungs-Datenwort zusammenzufassen, dieses mit je einem der ersten Aufbereitungs-Datenwörter 1,0,1,1 und 1,1,1,1 (entsprechend derselben Reihenfolge) zu vergleichen, und bei Übereinstimmung mit einem dieser Aufbereitungs-Datenwörter ein erstes logisches Steuersignal 1 dem Eingang 144 des Rechners 140 zuzuführen. Auf ähnliche Weise ist die Wirkung der Kombination der UND-Torschaltung 106, der Invertierer 44 und 54 und der entsprechenden Verbindungen, dasselbe Erkennungs-Datenwort zu bilden, es mit einem zweiten Aufbereitungs-Datenwort 0,0,1,1 zu vergleichen, und bei Übereinstimmung ein zweites logisches Steuersignal 1 dem Eingang 146 des Rechners 140 zuzuführen. Auf ähnliche Weise ist die Wirkung der Kombination der UND-Torschaltung 107, des Invertierers 64 und der entsprechenden Verbindungen, dasselbe Erkennungs-Datenwort mit je einem der Eichungs-Datenwörter 1,1,0,1 oder 0,1,0,1 oder 1,0,0,1 oder 0,0,0,1 zu vergleichen, und bei Übereinstimmung mit einem dieser Eichungs-Datenwörter ein den Eichungsvorgang bezeichnendes logisches Steuersignal 1 dem Eingang 147 des Rechners 140 zuzuführen.

Wie leicht ersichtlich ist, bedeutet das Erscheinen des ersten Steuersignals, daß in der Meßflüssigkeit das Verhältnis der Anzahl Erythrozyten zur Anzahl Thrombozyten ziemlich groß ist, eine Mindestanzahl Teilchen überhaupt vorhanden ist und die Leitfähigkeit ansehnlich ist, während die Anzahl Erythrozyten beliebig sein darf: dies entspricht also dem ersten Aufbereitungszustand (Blutverdünnung 1 : 80 000). Das Erscheinen des zweiten Steuersignals bedeutet, daß sowohl das Verhältnis der Anzahl Erythrozyten zur Anzahl Thrombozyten als auch die Anzahl Erythrozyten ziemlich klein ist jedoch eine Mindestanzahl Teilchen überhaupt vorhanden ist und die Leitfähigkeit ansehnlich ist: dies entspricht dem zweiten Aufbereitungszustand (Zentrifugation der Blutprobe und Verdünnung 1 : 2200 des überstehenden Plasmas). Das Erscheinen des den Eichungsvorgang bezeichnenden Steuersignals bedeutet, daß in der Meßflüssigkeit keine hinreichende Anzahl Teilchen, jedoch hohe Leitfähigkeit vorhanden ist. Das Verhältnis der Anzahl Erythrozyten zur Anzahl Thrombozyten sowie die Anzahl Erythrozyten dürfen beliebig sein: dies entspricht also dem Eichzustand, in welchem die Meßflüssigkeit aus reiner isotonischer Plasmaersatzlösung besteht.

Der Rechner 140 ist auf dem Fachmann naheliegende Weise zur Ausübung der folgenden Funktionen ausgebildet oder programmiert. Bei Erhalt des den Eichungsvorgang bezeichnenden Steuersignals auf dem Steuereingang 147 wird der auf dem Dateneingang 143 erhaltene Meßwert gespeichert, um als Basiswert von später erhaltenen, Blutproben entsprechenden Meßwerten abgezogen zu werden. Bei Erhalt des ersten Steuersignals auf dem Steuereingang 144 wird von dem auf dem Dateneingang 143 erhaltenen Meßwert der gespeicherte Basiswert abgezogen, worauf die erhaltene Differenz mit einem Koeffizienten multipliziert wird, der dem Betrieb der Meßvorrichtung 4 und dem Verdünnungsverhältnis 1 : 80 000 entspricht und das Resultat der Teilchenzählung in dem den Thrombozyten entsprechenden Kanal der Meßvorrichtung 4 in Anzahl Teilchen pro µl Blut umsetzt; das in dieser Einheit ausgedrückte Resultat wird angezeigt oder gedruckt. Bei Erhalt des zweiten Steuersignals auf dem Steuereingang 146 erfolgt derselbe Vorgang, mit dem Unterschied, daß der Koeffizient nun dem Verdünnungsverhältnis 1 : 2200 entspricht.

Das beschriebene Verfahren kann auch mit

anderen Vorrichtungen durchgeführt werden. Unter anderen Möglichkeiten können der Rechner 140, die UND-Torschaltungen 104, 106, 107 und die Invertierer 44, 54 zu einer einzigen Recheneinheit zusammengefaßt werden, welche die Funktionen dieser Vorrichtung ausübt. Wenn die Meßwerte an den Ausgängen 41, 51, 71 alle in digitaler Form vorliegen, ist es auch möglich, die Funktionen der Vergleicher 43, 53, 63, 73 sowie des Quotientenbildners 411 und des Addierwerkes 611 in die Recheneinheit aufzunehmen, so daß die drei Meßwerte direkt einer entsprechend programmierten Recheneinheit zugeführt werden; das Verfahren wird dann in dieser Recheneinheit durchgeführt.

Eine verallgemeinerte Form des Verfahrensablaufes wird in Fig. 3 schematisch dargestellt. Es sei mindestens ein Kennwert P aus vorbestimmten Kombinationen von Meßwerten $M_1$, $M_2 \dots M_p$ unter Verwendung von geeigneten Koeffizienten a, b, ... g, h zu errechnen. Ferner sei zwischen mehreren vorbestimmten möglichen Aufbereitungszuständen $A_1, A_2 \dots A_n$ und einem Eichzustand E zu unterscheiden. Beispielsweise gilt im ersten Aufbereitungszustand

$$P = a \cdot M_1 + b \cdot M_2,$$

im zweiten Aufbereitungszustand $P = h \cdot M_p$, während P im Eichzustand einen vorbestimmten Wert $P = P_E$ einzunehmen hat. Die eindeutige Unterscheidung zwischen den Aufbereitungszuständen sei nur durch Heranziehen von zusätzlichen Meßwerten $M_q \dots M_w$ möglich, während die Unterscheidung zwischen dem Eichungsvorgang und einer Reinigung der Meßvorrichtungen das Heranziehen eines weiteren Meßwertes $M_x$ erfordere. Es werden also, wie in Fig. 3 schematisch dargestellt, Meßwerte $M_1 \dots M_x$ gemessen. Die Meßwerte $M_1 \dots M_p$ werden einem Rechner R zugeführt, um darin zu einem Wert des Kennwertes P verarbeitet zu werden. Die Meßwerte $M_1 \dots M_x$ werden auf geeignete Weise zur Bildung von Hilfswerten $H_1, H_2 \dots H_t$, $H_u$ untereinander kombiniert; als Beispiel wurde dargestellt, daß $H_1$ aus $M_1$, $H_2$ aus $M_2$ und $M_p$, $H_t$ aus $M_2$, $M_p$, $M_q$ und $M_w$, und schließlich $H_u$ aus $M_q$ und $M_x$ hergeleitet werden (es gilt beispielsweise

$$H_1 = M_1,$$
$$H_2 = M_2/M_p,$$
$$H_t = M_2 + M_p + M_q + M_w \text{ und}$$
$$H_u = (M_q - M_x)/M_x).$$

Jedem Hilfswert $H_1, H_2 \dots H_t, H_u$ wird ein vorbestimmter Schwellenwert $S_1, S_2 \dots S_t, S_u$ zugeordnet; es wird im nachstehenden erläutert, wie die Schwellenwerte zu setzen sind. Zwischen jedem Hilfswert und dem zugeordneten Schwellenwert erfolgt je ein Vergleich $V_1, V_2 \dots V_t, V_u$. Als Ergebnis des Vergleiches wird je ein zugeordnetes Erkennungssignal $K_1, K_2 \dots K_t, K_u$ erzeugt, das beispielsweise den Istzustand 1 einnimmt, wenn der Hilfswert den Schwellenwert überschreitet, und im gegenteiligen Fall den Istzustand 0 einnimmt. Die Istzustände der Erkennungssignale $K_1, K_2 \dots K_t, K_u$ werden dann in einer bestimmten Anordnung oder Reihenfolge, beispielsweise in der Reihenfolge der Indizes 1, 2 ... t, u, zu einem Datenwort zusammengefaßt, das als Erkennungs-Datenwort DK dient. In derselben Anordnung oder Reihenfolge werden für jeden der möglichen Aufbereitungszustände sowie für den Eichzustand entsprechende vorbestimmte Sollzustände der Erkennungssignale zu einem Datenwort zusammengefaßt, das als jeweiliges Aufbereitungs-Datenwort $DA_1$, $DA_2 \dots DA_n$ sowie DE dient, wobei DE dem Eichzustand zugeordnet ist; es wird im nachstehenden erläutert, wie die Sollwerte zu setzen sind.

Zur Festsetzung der Schwellenwerte und der Sollzustände wird davon ausgegangen, daß bekannt ist, welche Art von Proben — z. B. menschliches Blut, Abwasser von Kläranlagen usw. — zu untersuchen ist, so daß auch bekannt ist, welche Eigenschaften der Proben immer innerhalb einer gegebenen Spannweite vorkommen und sich mit einem gegebenen Variationskoeffizienten um einen gegebenen Mittelwert häufen, und für welche Eigenschaften dies nicht zutrifft. Beispielsweise ist bekannt, daß im menschlichen Blut etwa 4 bis 6 Millionen Erythrozyten pro µl und etwa 120 bis 180 g/l Hämoglobin vorhanden ist, während die Konzentration der Thrombozyten meist zwischen 150 000 und 350 000 pro µl liegt, jedoch auch bis fast auf Null sinken kann; es ist auch bekannt, daß die spezifische Leitfähigkeit von üblichen Plasmaersatzlösungen meist zwischen 1,5 und 2 $Ohm^{-1} \cdot m^{-1}$ liegt. Zur Bildung von Hilfswerten werden also nur solche Meßwerte herangezogen, deren Größenordnung aufgrund der Eigenschaften der Proben und aufgrund der einzelnen möglichen Aufbereitungszustände bekannt ist. Bei einer gegebenen Art von Proben — z. B. bei menschlichem Blut — wird überlegt, welche aus jeweils einer bestimmten Kombination von Meßwerten gebildeten Hilfswerte in gut unterscheidbare Größenordnungen fallen, je nachdem sich die Probe im einen oder im anderen der Aufbereitungszustände befindet oder ein Eichzustand vorliegt. Beispielsweise geht aus dem vorangehenden hervor, daß in einer 1 : 80 000 verdünnten Blutprobe etwa 50 bis 75 Erythrozyten pro µl vorhanden sind, während in einem durch Zentrifugation von Blut erhaltenen Plasma nach einer 1 : 2200-Verdünnung höchstens etwa 15 Erythrozyten pro µl enthalten sind; zur Unterscheidung zwischen diesen beiden Aufbereitungszuständen kann also als Hilfswert der Meßwert für die Anzahl Erythrozyten verwendet werden, und der zugeordnete Schwellenwert wird dann gleich dem Meßwert gesetzt, der einer Meßflüssigkeit mit zwischen 15 und 50 Teilchen pro µl entspricht, zweckmäßigerweise einer Meßflüssigkeit etwa in der Mitte dieser Spanne, also mit 30 Teilchen pro µl. Allgemeiner wird jedem Hilfswert ein solcher Schwellenwert

zugeordnet, der die Aufbereitungszustände und den Eichzustand eindeutig in zwei Klassen teilt: die eine Klasse enthält Aufbereitungszustände und eventuell den Eichzustand, bei denen der Hilfswert eindeutig größer ist als der Schwellenwert; die andere Klasse enthält die übrigen Zustände; kein Hilfswert liegt dem Schwellenwert so nahe, daß die Zuordnung der Zustände zu den Klassen mit Unsicherheit behaftet wäre. Zahlenmäßige Beispiele sind bereits im vorangehenden gegeben worden.

Die Sollzustände der Erkennungssignale sind jeweils gleich denjenigen Istzuständen derselben Erkennungssignale, die erhalten werden, wenn eine Meßflüssigkeit analysiert wird, die entweder dem Eichzustand oder einem der vorbestimmten möglichen Aufbereitungszustände einer im nachstehenden definierten Normprobe entspricht. Eine äquivalente Formulierung ist, daß jeweils eines der Aufbereitungs-Datenwörter $DA_1$, $DA_2$ ... $DA_n$, DE gleich dem Erkennungs-Datenwort ist, das bei der Analyse einer Meßflüssigkeit erhalten wird, wenn diese Meßflüssigkeit entweder eine Normprobe in je einem der vorbestimmten möglichen Aufbereitungs-Zustände $A_1$, $A_2$ ... $A_n$ enthält oder im Eichzustand E vorliegt. Als Normprobe ist hier eine solche hypothetische Probe zu verstehen, bei welcher jede Eigenschaft, die über Meßwerte zur Bildung von Hilfswerten herangezogen wird, gleich dem statistischen Mittelwert für die betreffende Eigenschaft ist; Eigenschaften bzw. Meßwerte, die nicht zur Bildung von Hilfswerten herangezogen werden, sind hier nicht von Belang. Beispielsweise enthält eine Normprobe Blut pro µl 5 Millionen Erythrozyten, von denen nach einer Hämolyse genau 20 000 übrigbleiben und in denen gesamthaft 150 µg Hämoglobin enthalten ist sowie 250 000 Thrombozyten; eine Norm-Trägerflüssigkeit weist eine spezifische Leitfähigkeit von 1,75 $Ohm^{-1} \cdot m^{-1}$ auf, sie enthält kein Hämoglobin, jedoch 1 Teilchen µl. Selbstverständlich ist es nicht nötig, die Sollzustände durch Messung einer Normprobe zu bestimmen: zur Festsetzung der Sollzustände genügen die Kenntnis des Analysensystems, der Eigenschaften der hypothetischen Normprobe und der festgesetzten Schwellenwerte.

Im Endergebnis bezeichnet jedes Aufbereitungs-Datenwort sowie das Eichungs-Datenwort eindeutig nur einen Aufbereitungszustand oder den Eichzustand. Es kann allerdings dabei vorkommen, daß in gewissen dieser Wörter einer oder mehrere der Sollzustände einen beliebigen logischen Zustand 1 oder 0 einnehmen dürfen, was aber die Eindeutigkeit der Bezeichnung nicht beeinträchtigt: bei dem im Zusammenhang mit Fig. 2 beschriebenen Beispiel lautet das Eichungs-Datenwort x,x,0,1 mit x gleich 1 oder 0.

Im weiteren Ablauf des Verfahrens wird nun das Erkennungs-Datenwort DK mit jedem Aufbereitungs-Datenwort $DA_1$, $DA_2$ ... $DA_n$ und mit dem Eichungs-Datenwort DE verglichen; jedem dieser Vergleiche $VD_1$, $VD_2$ ... $VD_n$, $VD_E$ entspricht bei Übereinstimmung je ein logisches Steuersignal $ST_1$, $ST_2$... $ST_n$, $ST_E$, das den entsprechenden Aufbereitungszustand bezeichnet. Aus dem vorangehenden ergibt sich, daß die Übereinstimmung des Erkennungs-Datenwortes mit einem der Aufbereitungs-Datenwörter oder mit dem Eichungs-Datenwort die Übereinstimmung mit den anderen Wörtern ausschließt, so daß jederzeit nur eines der Steuersignale $ST_1$, $ST_2$ ... $ST_n$, $ST_E$ erzeugt werden kann. Dieses Steuersignal wird dem Rechner R zugeführt, um darin die Errechnung des Kennwertes P aus den zugeführten Meßwerten $M_1$, $M_2$ ... $M_p$ unter Verwendung der geeigneten Koeffizienten aus der Liste a, b ... g, h auszulösen; beispielsweise löst das Steuersignal $ST_1$ die Errechnung von P gemäß $P = a \cdot M_1 + b \cdot M_2$ aus, während das Steuersignal $ST_2$ die Errechnung von P gemäß $P = h \cdot M_p$ auslöst. Selbstverständlich könnten auch mehrere Kennwerte aus je einer vorbestimmten Kombination von Meßwerten und Koeffizienten errechnet werden, wie beispielsweise im Zusammenhang mit Fig. 1 für den zweiten Aufbereitungszustand beschrieben wurde. Das den Eichungsvorgang bezeichnende Steuersignal $ST_E$ löst im Rechner eine besondere Errechnung aus, bei welcher die entsprechenden Koeffizienten aus der Liste a, b ... g, h auf den geeigneten Wert gesetzt werden, um den Kennwert P auf den Eichwert $P = P_E$ zu bringen; im Zusammenhang mit Fig. 1 und 2 wurde dieser Eichwert $P_E$ als Basiswert bezeichnet. Die entsprechende Ausbildung oder Programmierung des Rechners R ist dem Fachmann naheliegend, sie braucht hier nicht näher beschrieben zu werden.

Mit dem beschriebenen Verfahren wird erreicht, daß ein Analysensystem selbstanpassend gesteuert wird, derart, daß die Errechnung des oder der Kennwerte einer zu untersuchenden Probe ohne Dazutun einer Bedienungsperson geeicht und dem Aufbereitungszustand der Probe entsprechend durchgeführt wird. Sofern die Probe in einem der vorgesehenen möglichen Aufbereitungszustände vorliegt, sind also Bedienungsfehler völlig ausgeschlossen.

**Patentansprüche**

1. Verfahren zur Analyse einer Meßflüssigkeit in einem Analysensystem, das mit Meßvorrichtungen zur Lieferung je eines Meßwertes für je eine bestimmte Eigenschaft der Meßflüssigkeit versehen ist, wobei die Meßflüssigkeit im wesentlichen aus einer Trägerflüssigkeit und einer darin in einem Aufbereitungszustand enthaltenen Probe besteht und der Aufbereitungszustand einer aus einer Anzahl dem Analysensystem zugeordneter vorbestimmter Aufbereitungszustände ist, und wobei mindestens ein Kennwert der Probe in einem Rechner aus mindestens einem der Meßwerte sowie mindestens einem dem Kennwert und dem Aufbereitungszustand zugeordneten Koeffizienten errechnet wird, gekennzeichnet durch folgende Verfahrensschritte:

— aus mindestens je einem der von ausgewählten Meßvorrichtungen gelieferten Meßwerte wird je einer von mehreren Hilfswerten gebildet;
— jeder Hilfswert wird mit einem vorbestimmten zugeordneten Schwellenwert verglichen, und es wird ein dem Ergebnis des Vergleiches zugeordnetes binäres Erkennungssignal erzeugt;
— die Erkennungssignale, in einer bestimmten Anordnung zu einem Erkennungs-Datenwort zusammengefaßt, werden verglichen mit für jeden der möglichen Aufbereitungszustände vorgegebenen Aufbereitungs-Datenwörtern, und bei Übereinstimmung des Erkennungs-Datenwortes mit einem Aufbereitungs-Datenwort wird ein den entsprechenden Aufbereitungszustand bezeichnendes logisches Steuersignal erzeugt;
— dieses Steuersignal wird dem Rechner zugeführt, um darin die Errechnung des Kennwertes auszulösen, welcher dem durch das Steuersignal bezeichneten Aufbereitungszustand entspricht.

2. Verfahren nach Anspruch 1 zur Blutanalyse in einem Analysensystem, das mindestens mit einer Meßvorrichtung zur Zählung von in der Meßflüssigkeit suspendierten Teilchen und einer Meßvorrichtung zur fotometrischen Messung eines Hämoglobingehaltes in der Meßflüssigkeit versehen ist, wobei die Meßflüssigkeit im wesentlichen aus einer isotonischen Plasmaersatzlösung und einer darin in einem von zwei Aufbereitungszuständen enthaltenen Blutprobe besteht und von diesen beiden Aufbereitungszuständen der erste einer höheren Verdünnung der Blutprobe in der Lösung und der zweite einer niedrigeren Verdünnung der Blutprobe in der Lösung unter Zugabe eines Hämolysiermittels entspricht, gekennzeichnet durch folgende Verfahrensschritte:

— es werden ein erster Hilfswert dem Ergebnis der Teilchenzählung und ein zweiter Hilfswert dem Ergebnis der fotometrischen Messung gleichgesetzt;
— der erste Hilfswert wird verglichen mit einem zugeordneten ersten Schwellenwert, der im Bereich von 5 bis 50% des mit einer physiologisch normalen Blutprobe im ersten Aufbereitungszustand zu erwartenden Ergebnisses der Teilchenzählung vorbestimmt wird, und es wird ein erstes Erkennungssignal erzeugt, dessen Zustand 1 oder 0 ist, je nachdem der erste Hilfswert größer oder kleiner ist als der erste Schwellenwert;
— der zweite Hilfswert wird verglichen mit einem zugeordneten zweiten Schwellenwert, der im Bereich von 5 bis 50% des mit einer physiologisch normalen Blutprobe im zweiten Aufbereitungszustand zu erwartenden Ergebnisses der fotometrischen Messung vorbestimmt wird, und es wird ein zweites Erkennungssignal erzeugt, dessen Zustand 1 oder 0 ist, je nachdem der zweite Hilfswert größer oder kleiner ist als der zweite Schwellenwert;
— wenn das Erkennungs-Datenwort aus der Zusammenfassung der Zustände 1 für das erste Erkennungssignal und 0 für das zweite Erkennungssignal besteht, wird ein erstes Steuersignal erzeugt und mindestens einem Rechner zugeführt, um darin eine Errechnung der Erythrozytenkonzentration aus dem Ergebnis der Teilchenzählung sowie aus einem zugeordneten Koeffizienten auszulösen;
— wenn das Erkennungs-Datenwort den Zustand 1 für das zweite Erkennungssignal enthält, wird ein zweites Steuersignal erzeugt und mindestens einem Rechner zugeführt, um darin eine Errechnung der Leukozytenkonzentration aus dem Ergebnis der Teilchenzählung sowie aus einem zugeordneten Koeffizienten und eine Errechnung der Hämoglobinkonzentration aus dem Ergebnis der fotometrischen Messung sowie aus einem zugeordneten Koeffizienten auszulösen.

3. Verfahren nach Anspruch 1 zur Blutanalyse in einem Analysensystem, das mindestens mit einer Meßvorrichtung zum Analysieren von in der Meßflüssigkeit suspendierten Teilchen auf deren Größenklasse und Anzahl pro Größenklasse versehen ist, wobei die Meßflüssigkeit im wesentlichen aus einer isotonischen Plasmaersatzlösung und einer darin in einem von zwei Aufbereitungszuständen enthaltenen Blutprobe besteht und von diesen beiden Aufbereitungszuständen der erste einer höheren Verdünnung der Blutprobe in der Lösung und der zweite einer niedrigeren Verdünnung eines aus der Blutprobe abgeschiedenen Plasma-Überstandes entspricht, gekennzeichnet durch folgende Verfahrensschritte:

— es wird das Verhältnis des Ergebnisses der Teilchenzählung in der den Erythrozyten entsprechenden Größenklasse zum Ergebnis der Teilchenzählung in der den Thrombozyten entsprechenden Größenklasse errechnet;
— es werden ein erster Hilfswert dem genannten Verhältnis und ein zweiter Hilfswert dem Ergebnis der Teilchenzählung in der den Erythrozyten entsprechenden Größenklasse gleichgesetzt;
— der erste Hilfswert wird verglichen mit einem zugeordneten ersten Schwellenwert, der im Bereich von 5 bis 50% des mit einer physiologisch normalen Blutprobe im ersten Aufbereitungszustand zu erwartenden Wertes des genannten Verhältnisses vorbestimmt wird, und es wird ein erstes Erkennungssignal erzeugt, dessen Zustand 1

oder 0 ist, je nachdem der erste Hilfswert größer oder kleiner ist als der erste Schwellenwert;

- der zweite Hilfswert wird verglichen mit einem zugeordneten zweiten Schwellenwert, der im Bereich von 5 bis 50% des mit einer physiologisch normalen Blutprobe im zweiten Aufbereitungszustand zu erwartenden Ergebnisses der Teilchenzählung in der den Erythrozyten entsprechenden Größenklasse vorbestimmt wird, und es wird ein zweites Erkennungssignal erzeugt, dessen Zustand 1 oder 0 ist, je nachdem der zweite Hilfswert größer oder kleiner ist als der zweite Schwellenwert;
- wenn das Erkennungs-Datenwort den Zustand 1 für das erste Erkennungssignal enthält, wird ein erstes Steuersignal erzeugt, und wenn das Erkennungs-Datenwort aus der Zusammenfassung der Zustände 0 für beide Erkennungssignale besteht, wird ein zweites Steuersignal erzeugt;
- jedes dieser Steuersignale löst im Rechner eine Errechnung der Thrombozytenkonzentration aus dem Ergebnis der Teilchenanalyse in der den Thrombozyten entsprechenden Größenklasse sowie aus einem zugeordneten Koeffizienten aus, wobei dem ersten Steuersignal der der höheren Verdünnung entsprechende Koeffizient und dem zweiten Steuersignal der der niedrigeren Verdünnung entsprechende Koeffizient zugeordnet ist.

4. Verfahren nach Anspruch 1, wobei das Analysensystem zusätzlich mit einer Meßvorrichtung zur Messung der Leitfähigkiet der Meßflüssigkeit versehen ist, gekennzeichnet durch folgende Verfahrensschritte:

- das Ergebnis der Leitfähigkeitsmessung wird verglichen mit einem zugeordneten Eichungs-Schwellenwert, der im Bereich von 5 bis 50% des mit der reinen Trägerflüssigkeit zu erwartenden Ergebnisses der Leitfähigkeitsmessung vorbestimmt wird, und es wird ein binäres Eichungssignal erzeugt, dessen Zustand 1 oder 0 ist, je nachdem das Ergebnis der Leitfähigkeitsmessung größer oder kleiner ist als der Eichungs-Schwellenwert;
- die Erkennungssignale und das Eichungssignal, in einer bestimmten Anordnung zu einem erweiterten Erkennungs-Datenwort zusammengefaßt, werden verglichen mit für jeden der möglichen Aufbereitungszustände vorgegebenen erweiterten Aufbereitungs-Datenwörtern sowie mit mindestens einem der reinen Trägerflüssigkeit entsprechenden vorgegebenen Eichungs-Datenwort, und bei Übereinstimmung des erweiterten Erkennungs-Datenwortes mit einem Eichungs-Datenwort wird ein den Eichungsvorgang bezeichnendes logisches Steuersignal erzeugt;
- dieses Steuersignal wird dem Rechner zugeführt, um darin einen Eichungsvorgang auszulösen.

5. Verfahren nach den Ansprüchen 3 und 4, gekennzeichnet durch folgende zusätzliche Verfahrensschritte:

- es wird die Summe des Ergebnisses der Teilchenzählung in der den Erythrozyten entsprechenden Größenklasse und des Ergebnisses der Teilchenzählung in der den Thrombozyten entsprechenden Größenklasse errechnet;
- es wird ein dritter Hilfswert dieser genannten Summe gleichgesetzt;
- der dritte Hilfswert wird verglichen mit einem zugeordneten dritten Schwellenwert, der im Bereich von 5 bis 50% des mit einer physiologisch normalen Blutprobe im ersten Aufbereitungszustand zu erwartenden Ergebnisses der Errechnung der genannten Summe vorbestimmt wird, und es wird ein drittes Erkennungssignal erzeugt, dessen Zustand 1 oder 0 ist, je nachdem der dritte Hilfswert größer oder kleiner ist als der dritte Schwellenwert.

## Claims

1. Process for analysing a measuring fluid in an analysis system which is provided with measuring devices for supplying a measurement value for each specific property of the measuring fluid, wherein the measuring fluid substantially comprises a carrier fluid and a sample in a pre-processed state, and the pre-processed state is one of a plurality of specific pre-processed states associated with the analysis system, and wherein at least one characteristic value of the sample is calculated in a computer from at least one of the measurement values and at least one coefficient associated with the characteristic value and the pre-processed state, characterized by the following process steps:

- from at least one of the measurement values supplied by selected measuring devices, one of several auxiliary values is formed;
- each auxiliary value is compared with a predetermined associated threshold value, and a binary recognition signal associated with the result of the comparison is produced;
- the recognition signals, combined in a specific arrangement to form a recognition-data word, are compared with predetermined pre-processed-data words for each of the possible pre-processed states, and when the recognition-data word agrees with a pre-processed-data word a logical control signal designating the corresponding pre-processed state is produced;
- this control signal is supplied to the

computer in order to release therein the calculation of the characteristic value, which corresponds to the pre-processed state designated by the control signal.

2. Process according to claim 1 for blood analysis in an analysis system which is provided with at least one measuring device for counting particles suspended in the measuring fluid and a measuring device for the photometric measuring of a haemoglobin content in the measuring fluid, wherein the measuring fluid substantially comprises an isotonic plasma substitute solution and a blood sample contained in it in one of two pre-processed states, and of these two pre-processed states the first corresponds to a higher dilution of the blood sample in the solution and the second to a lower dilution of the blood sample in the solution when a haemolysing agent is added, characterized by the following process steps:

- a first auxiliary value is equated with the result of the particle count and a second auxiliary value is equated with the result of the photometric measurement;
- the first auxiliary value is compared with an associated first threshold value which is predetermined in the range from 5% to 50% of the result of the particle count that is to be expected with a physiologically normal blood sample in the first pre-processed state, and a first recognition signal is produced whose state is 1 or 0 according to whether the first auxiliary value is larger or smaller than the first threshold value;
- the second auxiliary value is compared with an associated second threshold value which is predetermined in the range from 5% to 50% of the result of the photometric measurement that is to be expected in the second pre-processed state with a physiologically normal blood sample, and a second recognition signal is produced whose state is 1 or 0 according to whether the second auxiliary value is larger or smaller than the second threshold value;
- if the recognition-data word is comprised of the combination of the states 1 for the first recognition signal and 0 for the second recognition signal, a first control signal is produced and is supplied to at least one computer in order to release therein a calculation of the erythrocyte concentration from the result of the particle count and from an associated coefficient;
- if the recognition-data word contains the state 1 for the second recognition signal, a second control signal is produced and is supplied to at least one computer in order to release therein a calculation of the leukocyte concentration from the result of the particle count and from an associated coefficient and a calculation of the haemoglobin concentration from the result of the photometric measurement and from an associated coefficient.

3. Process according to claim 1 for blood analysis in an analysis system which is provided with at least one measuring device for analysing particles suspended in the measuring fluid according to their size class and number per size class, wherein the measuring fluid substantially comprises an isotonic plasma substitute solution and a blood sample contained therein in one of two pre-processed states, and of these two pre-processed states the first corresponds to a higher dilution of the blood sample in the solution and the second to a lower dilution of a plasma separated out on top of the blood sample, characterized by the following process steps:

- the ratio of the result of the particle in the size class corresponding to the erythrocytes to the result of the particle count in the size class corresponding to the thrombocytes is calculated;
- a first auxiliary value is equated to the abovementioned ratio and a second auxiliary value is equated to the result of the particle count in the size class corresponding to the erythrocytes;
- the first auxiliary value is compared to an associated first threshold value which is predetermined in the range 5% to 50% of the value of the abovementioned ratio to be expected with a physiologically normal blood sample in the first pre-processed state, and a first recognition signal is produced whose state is 1 or 0, according to whether the first auxiliary value is larger or smaller than the first threshold value;
- the second auxiliary value is compared to an associated second threshold value which is predetermined in the range of 5% to 50% of the result of the particle count in the size class corresponding to the erythrocytes that is to be expected with a physiologically normal blood sample in the second pre-processed state, and a second recognition signal is produced whose state is 1 or 0 according to whether the second auxiliary value is larger or smaller than the second threshold value;
- if the recognition-data word contains the state 1 for the first recognition signal, a first control signal is produced, and if the recognition-data word consists of the combination of the states 0 for both recognition signals, a second control signal is produced;
- each of these control signals releases in the computer a calculation of the thrombocyte concentration from the result of the particle analysis in the size class corresponding to the thrombocytes, and from an associated coefficient, whereby the coefficient corresponding to the higher dilution is associated

with the first control signal and the coefficient corresponding to the lower dilution is associated with the second control signal.

4. Process according to claim 1, wherein the analysis system is additionally provided with a measuring device for measuring the conductivity of the measuring fluid, characterized by the following process steps:

- the result of the conductivity measurement is compared to an associated calibration threshold value which is predetermined in the range of 5% to 50% of the result of the conductivity measurement that is to be expected with the pure carrier fluid, and a binary calibration signal is produced whose state is 1 or 0 according to whether the result of the conductivity measurement is larger of smaller than the calibration threshold value;
- the recognition signals and the calibration signal, combined in a specific arrangement to form an enlarged recognition-data word, are compared with predetermined enlarged pre-processed-data words for each of the possible pre-processed states and with at least one predetermined calibration-data word corresponding to the pure carrier fluid, and when the enlarged recognition-data word agrees with a calibration-data word there is produced a logical control signal designating the calibration process;
- this control signal is supplied to the computer so as to release therein a calibration process.

5. Process according to claims 3 and 4, characterized by the following additional process steps;

- the sum of the result of the particle count in the size class corresponding to the erythrocytes and the result of the particle count in the size class corresponding to the thrombocytes is calculated;
- a third auxiliary value is equated to this abovementioned sum;
- the third auxiliary value is compared with an associated third threshold value which is predetermined in the range 5% to 50% of the result of the calculation of the abovementioned sum that is to be expected with a physiologically normal blood sample in the first pre-processed state, and a third recognition signal is produced whose state is 1 or 0 according to whether the third auxiliary value is larger or smaller than the third threshold value.

**Revendications**

1. Procédé d'analyse d'un liquide de mesure d'un système d'analyse comportant des dispositifs pour fournir respectivement une grandeur de mesure pour chaque caractéristique déterminée du liquide de mesure, le liquide de mesure se composant essentiellement d'un liquide vecteur contenant un échantillon à l'état préparatoire et l'état préparatoire étant l'un des états préparatoires prédéterminés associés au système d'analyse, et au moins une grandeur caractéristique de l'échantillon étant calculée dans un calculateur à partir d'au moins l'une des grandeurs de mesure ainsi que d'au moins l'un des coefficients associés à la grandeur de mesure et à l'état préparatoire, procédé caractérisé par les phases opératoires suivantes:

- à partir d'au moins chacune des grandeurs de mesure fournies par les dispositifs de mesure choisis, on forme l'une des grandeurs auxiliaires,
- chaque grandeur auxiliaire est comparee à une valeur de seuil associée, predeterminee et on crée un signal de reconnaissance binaire associé au résultat de la comparaison,
- les signaux de reconnaissance sont réunis suivant une association prédéterminée pour former un mot de données de reconnaissance, ils sont comparés aux mots de données de préparation, prédéterminés associés à chacun des états préparatoires possibles et en cas de coïncidence du mot de données de reconnaissance et d'un mot de données préparatoire, on crée un signal de commande logique caractéristique de l'état préparatoire correspondant,
- on applique ce signal de commande à un calculateur pour y déclencher le calcul de la grandeur caractéristique qui correspond à l'état préparatoire désigné par le signal de commande.

2. Procédé selon la revendication 1 pour l'analyse sanguine dans un système d'analyse qui se compose d'au moins un dispositif de mesure pour le comptage de particules en suspension dans le liquide de mesure et d'un dispositif de mesure pour la mesure photométrique de la teneur en hémoglobine dans le liquide de mesure, le liquide de mesure se composant essentiellement d'une solution de remplacement de plasma isotonique et d'un échantillon sanguin qui est contenu suivant deux états préparatoires, et parmi ces deux états préparatoires, le premier correspond à une dilution plus élevée de l'échantillon sanguin dans la solution et le second a une dilution plus faible de l'échantillon sanguin dans la solution, avec addition d'un agent d'hémolyse, procédé caractérisé par les phases opératoires suivantes:

- on fixe une première grandeur auxiliaire correspondant au résultat du comptage de particules et une seconde grandeur auxiliaire correspondant au résultat de la mesure

photométrique,

- on compare la première grandeur auxiliaire à une première grandeur de seuil correspondante qui est fixée dans une zone correspondant à 5%—50% du résultat prévisible du comptage des particules pour un échantillon sanguin physiologiquement normal dans un premier état préparatoire et on crée un premier signal de reconnaissance dont l'état est égal à 1 ou à 0 suivant que la première grandeur auxiliaire est supérieure ou inférieure à cette première grandeur de seuil,
- on compare la seconde grandeur auxiliaire à une seconde grandeur de seuil correspondante qui se situe entre 5% et 50% du résultat prévisible de la mesure photométrique sur un échantillon sanguin physiologiquement normal pris dans un second état préparatoire, et on crée un second signal de reconnaissance dont l'état est égal à 1 ou à 0 suivant que la seconde grandeur auxiliaire est supérieure ou inférieure à la seconde grandeur de seuil,
- si le mot de données de reconnaissance se compose de la réunion des états 1 pour le premier signal de reconnaissance et 0 pour le second signal de reconnaissance, on crée un premier signal de commande et on l'applique à au moins un calculateur pour y calculer la concentration en érythrocyte à partir du résultat du comptage des particules et d'un coefficient correspondant,
- si le mot de données de reconnaissance contient l'état 1 pour le second signal de reconnaissance, on crée un second signal de commande et on l'applique à au moins un calculateur pour y effectuer le calcul de la concentration des leucocytes à partir du résultat du comptage des particules et d'un coefficient correspondant et pour calculer la concentration en hémoglobine à partir du résultat de la mesure photométrique et d'un coefficient correspondant.

3. Procédé selon la revendication 1 pour l'analyse sanguine dans un système d'analyse qui comporte au moins un dispositif de mesure pour analyser des particules en suspension dans un liquide de mesure d'après la classe de dimensions et le nombre par classe de dimensions, le liquide de mesure se composant essentiellement d'une solution de remplacement de plasma, isotonique et d'un échantillon sanguin qui est contenu suivant l'un des deux états préparatoire, et parmi ces deux états préparatoires le premier correspond à une dilution plus élevée de l'échantillon sanguin dans la solution et le second à une dilution plus faible d'un résidu de plasma séparé de l'échantillon sanguin, procédé caractérisé par les phases opératoires suivantes:

- on calcule le rapport du résultat du comptage des particules dans la classe des dimensions correspondant aux érythrocytes et du résultat du comptage des particules dans la classe des dimensions correspondant aux thrombocytes,
- on fixe une première grandeur auxiliaire égale au rapport mentionné et une seconde grandeur auxiliaire égale au résultat du comptage des particules dans la classe des dimensions correspondant aux érythrocytes,
- on compare la première grandeur auxiliaire à une première grandeur de seuil correspondante qui est comprise entre 5% et 50% de la grandeur prévisible du rapport mentionné, pour un échantillon sanguin physiologiquement normal dans un premier état préparatoire, et on crée un premier signal de reconnaissance dont l'état est égal à 1 ou à 0 suivant que la première grandeur auxiliaire est supérieure ou inférieure à la première grandeur de seuil,
- on compare la seconde grandeur auxiliaire à une seconde grandeur de seuil correspondante qui est comprise entre 5% et 50% du résultat prévisible du comptage de particules dans la classe des dimensions correspondant aux érythrocytes pour un échantillon sanguin physiologiquement normal pris dans un second état préparatoire, et on crée un second signal de reconnaissance dont l'état est égal à 1 ou 0 suivant que la seconde grandeur auxiliaire est supérieure ou inférieure à la seconde grandeur de seuil,
- si le mot de données de reconnaissance contient l'état 1 pour le premier signal de reconnaissance, on crée un premier signal de commande et si le mot de données de reconnaissance se compose des états 0 pour les deux signaux de reconnaissance, on crée un second signal de commande,
- chacun des signaux de commande déclenche dans le calculateur un calcul de la concentration en thrombocytes à partir du résultat de l'analyse des particules dans la classe des dimensions correspondant aux thrombocytes et d'un coefficient correspondant, le coefficient correspondant à la plus grande dilution étant associé au premier signal de commande alors que le coefficient correspondant à la dilution la plus faible est associé au second signal de commande.

4. Procédé selon la revendication 1, le système d'analyse comportant en outre un dispositif de mesure pour mesurer la conductibilité du liquide de mesure, procédé caractérisé par les phases opératoires suivantes:

- on compare le résultat de la mesure de la conductibilité à une grandeur de seuil de tarage correspondante qui est comprise entre 5% et 50% du résultat prévisible de la mesure de conductibilité pour un liquide vecteur pur, et on crée un signal binaire de tarage dont l'état est égal à 1 ou à 0 suivant que le résultat de la mesure de conducitibi-

lité est supérieur ou inférieur à la grandeur de seuil de tarage,

— les signaux de reconnaissance et le signal de tarage sont réunis suivant une combinaison prédéterminée pour former un mot de données de reconnaissance, élargi, on compare ce mot aux mots de données préparatoires élargis, prédéterminés de chacun des états préparatoires possibles, ainsi qu'à au moins l'un des mots de données de tarage prédéterminés correspondant au liquide vecteur pur et en cas de coïncidence du mot de données de reconnaissance élargi et d'un mot de données de tarage, on crée un signal de commande logique caractéristique de l'opération de tarage,

— on applique ce signal de commande au calculateur pour y déclencher une opération de tarage.

5. Procédé selon l'une quelconque des revendications 3 et 4, caractérisé par les phases opératoires supplémentaires suivantes:

— on calcule la somme des résultats du comptage des particules dans la classe des dimensions correspondant aux érythrocytes et du résultat du comptage des particules dans la classe des dimensions correspondant aux thrombocytes,

— on fixe une troisième grandeur auxiliaire égale à la somme mentionnée,

— on compare la troisième grandeur auxiliaire à une troisième grandeur de seuil correspondante mais qui est comporise entre 5% et 50% du résultat prévisible du calcul de la somme mentionnée pour un échantillon sanguin physiologiquement normal se trouvant dans le premier état préparatoire, et on crée un troisième signal de reconnaissance dont l'état est égal à 1 ou à 0 suivant que la troisième grandeur auxiliaire est supérieure ou inférieure à la troisième grandeur de seuil.

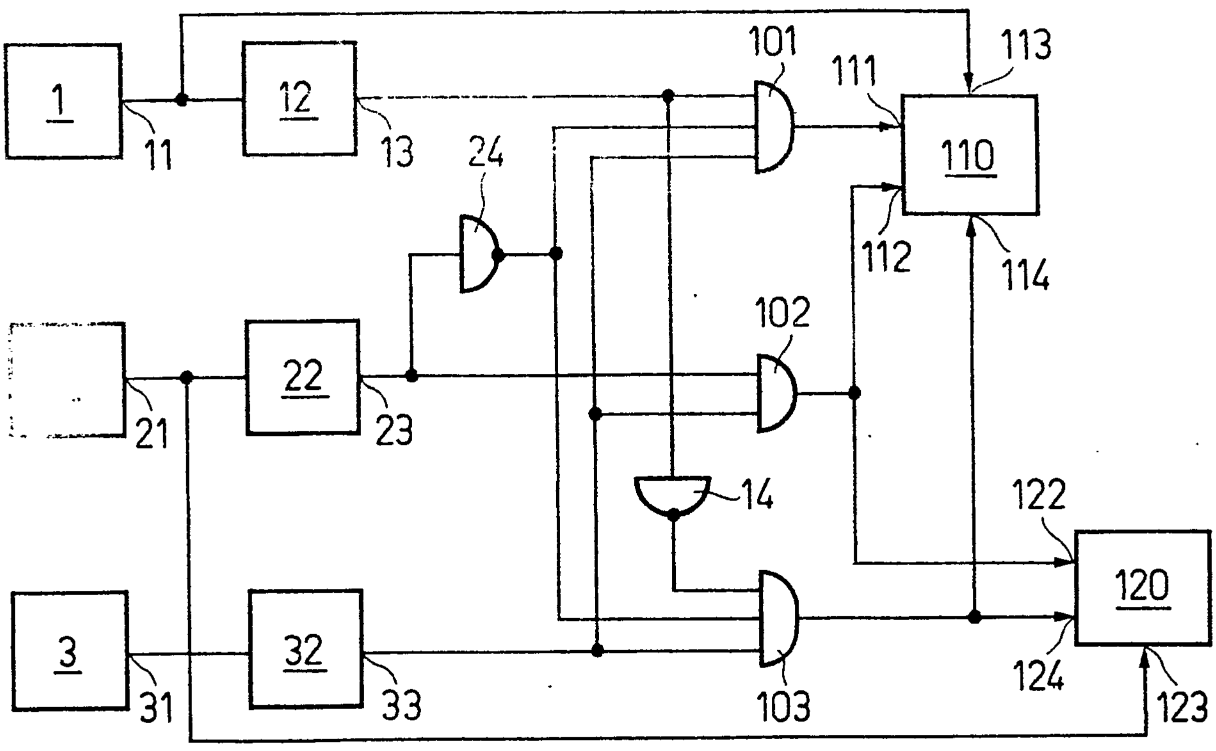
1
11
12
13
24
101
111
113
110
112
114
21
22
23
102
14
122
120
3
31
32
33
103
124
123

FIG. 1

FIG. 2

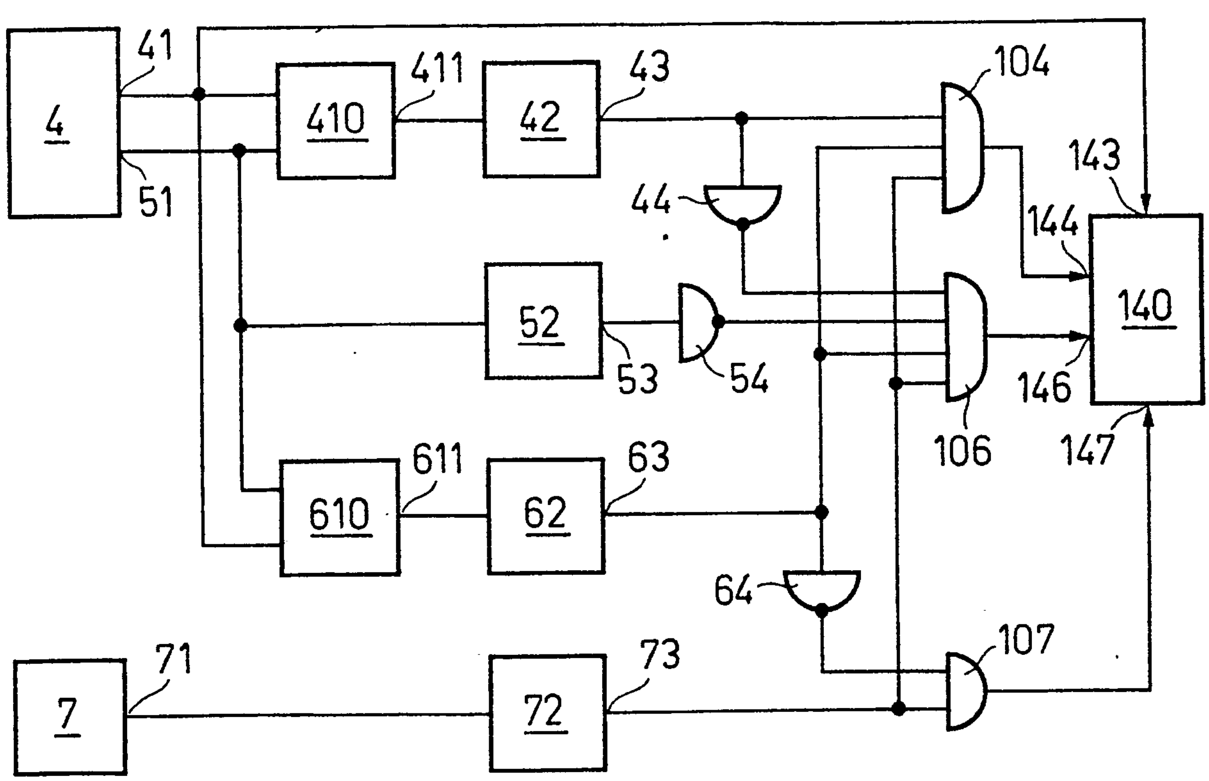
4
41
51
410
411
42
43
104
44
143
144
140
52
53
54
146
106
147
610
611
62
63
64
7
71
72
73
107

FIG. 3

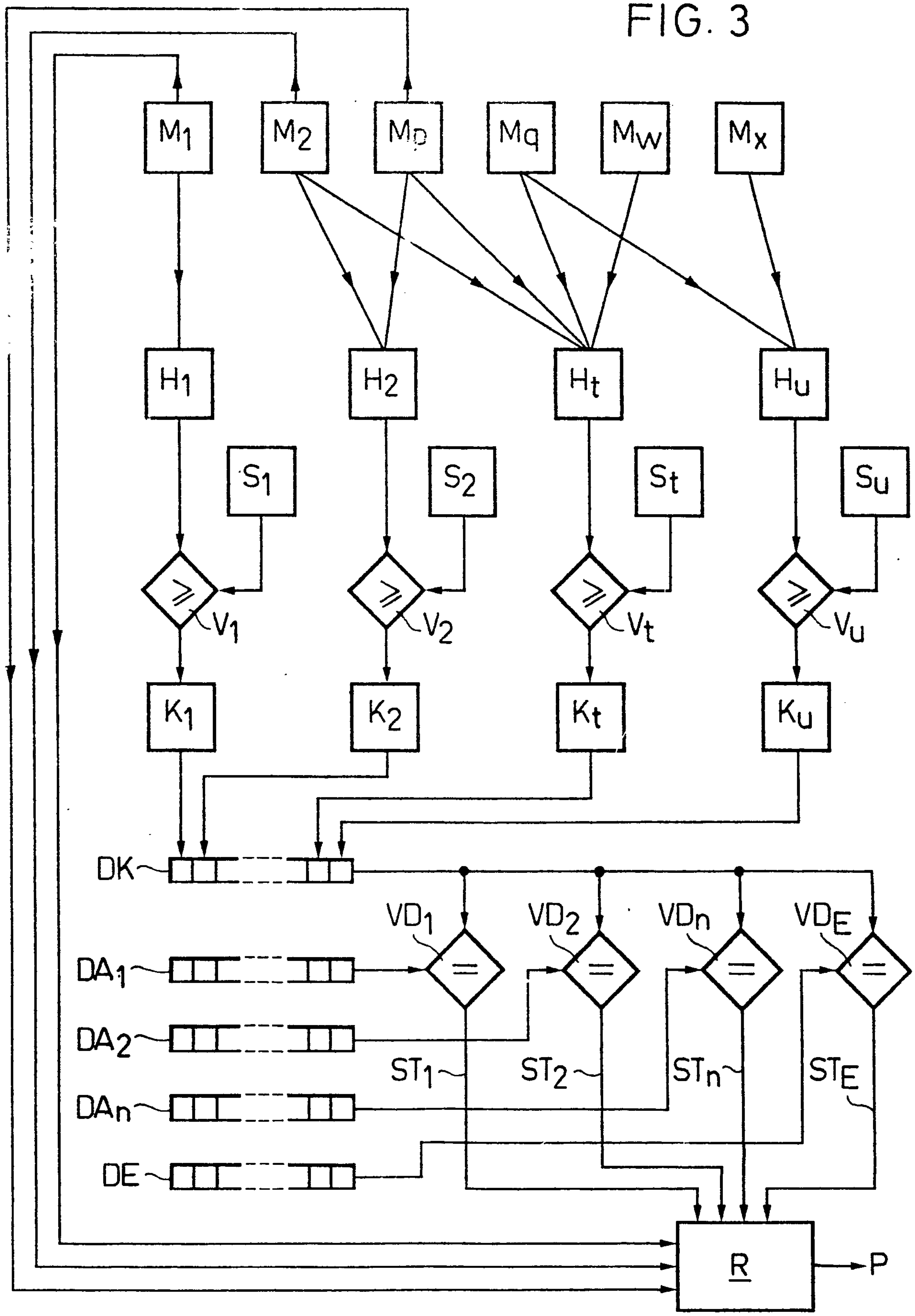
M1
M2
Mp
Mq
Mw
Mx
H1
H2
Ht
Hu
S1
S2
St
Su
V1
V2
Vt
Vu
K1
K2
Kt
Ku
DK
VD1
VD2
VDn
VDE
DA1
DA2
DAn
DE
ST1
ST2
STn
STE
R
P